# EUROPÄISCHE PATENTSCHRIFT

(11) **EP 3 171 750 B1**
(45) Veröffentlichungstag und Bekanntmachung des Hinweises auf die Patenterteilung: **17.04.2019**
(21) Anmeldenummer: 15739581.5
(22) Anmeldetag: 20.07.2015
(51) Int. Cl.: A47L 15/42, D06F 39/00, D06F 58/20

(54) **HAUSHALTSGERÄT MIT TROPFENERZEUGER**
DOMESTIC APPLIANCE HAVING A DROP GENERATOR
APPAREIL MÉNAGER AVEC GÉNÉRATEUR DE GOUTTES

(30) Priorität: 23.07.2014 DE 102014214341
(43) Veröffentlichungstag der Anmeldung: 31.05.2017
(73) Patentinhaber: BSH Hausgeräte GmbH, 81739 München (DE)
(72) Erfinder: EGLMEIER, Hans, 10587 Berlin (DE); SCHULZE, Ingo, 16341 Panketal (DE)
(86) Internationale Anmeldenummer: PCT/EP2015/066559
(87) Internationale Veröffentlichungsnummer: WO 2016/012404

(56) Entgegenhaltungen:
- EP-A1- 1 431 443
- WO-A1-2013/171109
- WO-A1-2014/023579

## Beschreibung

Die vorliegende Erfindung betrifft ein Haushaltsgerät mit einem Tropfenerzeuger zum Erzeugen eines Flüssigkeitstropfennebels.

Zur Erhöhung einer Reinigungseffizienz kann in modernen Haushaltsgeräten wie Waschmaschinen Flüssigkeitstropfennebel eingesetzt werden. Beim Einsatz von Flüssigkeitstropfennebel in Haushaltsgeräten ist die Verteilung der Tropfengrößen im erzeugten Flüssigkeitstropfennebel von grundlegender Bedeutung. Übliche Vorrichtungen zum Erzeugen von Flüssigkeitstropfennebel oder übliche Nebelgeneratoren erzeugen ein Spektrum von Tropfengrößen. Insbesondere große Tropfen schlagen sich im Betrieb des Haushaltsgerätes an Schaugläsern und am Behandlungsgut nieder, wo sie Flüssigkeitsflecken hinterlassen.

Die Offenlegungsschrift WO 2014/023579 A1 offenbart ein Haushaltsgerät mit einem Strömungsbehälter, einem an dem Strömungsbehälter angeordneten Tropfenerzeuger zum Erzeugen von Flüssigkeitstropfen aus einer Flüssigkeit in einem Strömungsbehälterbereich des Strömungsbehälters, einem Flüssigkeitstropfenauslass und einem gegenüber dem Flüssigkeitstropfenauslass angeordneten Strömungserzeuger zum Erzeugen einer Luftströmung zum Befördern der Flüssigkeitstropfen zum Flüssigkeitstropfenauslass.

Die Druckschrift JP2008194261A offenbart eine Zerstäubungseinheit zum Erzeugen von Nebel. Der Nebel besteht hauptsächlich aus nassem Nebel und weist eine Partikelgröße von 10-50 µm auf. Er wird über eine Entlüftungsöffnung durch Eintropfen des Nebels in ein Wasserrohr in eine Drehtrommel eingebracht.

Die Druckschrift JP2009082643A offenbart einen Wasch-Trockner vom Trommeltyp, der eine nebelerzeugende Zerstäubungseinheit und eine Lochung des Trommelbodens umfasst. Eine oder mehrere Radial- und Umfangsrippen sind an einem konzentrischen Kreis an der Rückseite des Trommelbodens angeordnet und im Fall übermäßig erzeugten Nebels fängt die Lochung des Trommelbodens übermäßigen Nebel ab und lässt abgefangenes und gesammeltes Wasser an der Rückseite des Trommelbodens entlang der Radial- und der Umfangsrippen in Richtung eines Wassertanks abfließen.

Die Druckschrift EP2462954A2 offenbart ein Plasmaerzeugungsgerät und ein Verfahren zur Sterilisation und Deodorisierung in Haushaltsgeräten. Ein Paar von Elektroden wird vorbereitet und Plasmaaustragung wird durch Anwenden einer bestimmten Spannung zwischen dem Paar von Elektroden durchgeführt.

Es ist die der Erfindung zugrunde liegende Aufgabe, ein effizientes Konzept zur Reduktion von großen Tropfen eines Flüssigkeitstropfennebels in einem Haushaltsgerät zu schaffen.

Diese Aufgabe wird durch den Gegenstand mit den Merkmalen nach dem unabhängigen Anspruch gelöst. Vorteilhafte Ausführungsformen der Erfindung sind Gegenstand der Figuren, der Beschreibung und der abhängigen Ansprüche.

Die Erfindung basiert auf der Erkenntnis, dass durch eine Verlängerung einer Wegstrecke zum Flüssigkeitstropfenauslass hin größere Flüssigkeitstropfen den Flüssigkeitstropfenauslass nicht erreichen, weil sie aufgrund der Gravitation vorher absinken. Dadurch erreichen nur Tropfen einer bestimmten Größe bzw. mit einem bestimmten maximalen Radius den Flüssigkeitstropfenauslass, da diese zusätzlich durch den Effekt der Stokes'schen Reibung länger in der Luft gehalten werden, während die größeren Tropfen in dem Strömungsbehälter verbleiben. Dadurch tritt ein besonders feiner Flüssigkeitstropfennebel aus dem Strömungsbehälter aus.

Gemäß einem Aspekt der Erfindung wird die Aufgabe durch ein Haushaltsgerät mit einem Strömungsbehälter, einem an dem Strömungsbehälter angeordneten Tropfenerzeuger zum Erzeugen von Flüssigkeitstropfen aus einer Flüssigkeit in einem Strömungsbehälterbereich des Strömungsbehälters, einem Flüssigkeitstropfenauslass und einem gegenüber dem Flüssigkeitstropfenauslass angeordneten Strömungserzeuger zum Erzeugen einer Luftströmung zum Befördern der Flüssigkeitstropfen zum Flüssigkeitstropfenauslass gelöst, wobei ein Abstand zwischen dem Strömungsbehälterbereich und dem Flüssigkeitstropfenauslass derart gewählt ist, dass eine erste Zeitspanne der Beförderung von Flüssigkeitstropfen zwischen dem Strömungsbehälterbereich und dem Flüssigkeitstropfenauslass gleich oder größer als eine zweite Zeitspanne des Absinkens eines Flüssigkeitstropfens einer vorbestimmten Tropfengröße entlang einer vorbestimmten Absinkstrecke ist. Dadurch wird beispielsweise der technische Vorteil erreicht, dass die Größe der Flüssigkeitstropfen in einem bereits erzeugten Flüssigkeitstropfennebel reduziert werden kann.

Unter einem Haushaltsgerät wird insbesondere ein Haushaltsgerät verstanden, das zur Haushaltsführung in Haushalten eingesetzt wird und insbesondere dazu dient Textilien wie Wäsche und Bekleidung oder auch Geschirr und Küchenartikel zu reinigen bzw. zu waschen. Das kann ein Haushaltsgerät sein wie beispielsweise ein Waschmaschine, ein Waschtrockner, ein Wäschetrockner oder eine Geschirrspülmaschine.

In einer weiteren vorteilhaften Ausführungsform entspricht die vorbestimmte Absinkstrecke dem Abstand zwischen einer Oberseite des Strömungsbehälters und einer Oberfläche der Flüssigkeit in dem Strömungsbehälter. Dadurch wird beispielsweise der technische Vorteil erreicht, dass der Strömungsbehälter in Abhängigkeit von der Anordnung des Strömungsbehälterbereichs einfach zu Dimensionieren und herstellbar ist.

In einer weiteren vorteilhaften Ausführungsform liegt die vorbestimmte Größe der Flüssigkeitstropfen in einem Bereich zwischen 1µm und 10µm und beträgt vorzugsweise 5µm. Dadurch wird beispielsweise der technische Vorteil erreicht, dass der Flüssigkeitstropfennebel mehr Homogenität und eine verbesserte Sichtbarkeit aufweist, was sich in einer homogeneren Befeuchtung des Behandlungsgutes niederschlägt. Insbesondere bleibt der Flüssigkeitstropfennebel erhalten, bis er vom Ort seiner Erzeugung bis zum Ort des Behandlungsgutes gelangt ist.

In einer weiteren vorteilhaften Ausführungsform ist der Tropfenerzeuger in dem Strömungsbehälterbereich insbesondere in oder am Boden des Strömungsbehälters angeordnet. Dadurch wird beispielsweise der technische Vorteil erreicht, dass der genaue Ort des Entstehens der Flüssigkeitstropfen in dem Strömungsbehälter vorbestimmbar ist.

In einer weiteren vorteilhaften Ausführungsform ist der Tropfenerzeuger ein Piezoschwinger mit einer Schwingfrequenz im Bereich zwischen 1MHz und 2 MHz, wobei die Schwingfrequenz vorzugsweise 1,6MHz beträgt. Dadurch wird beispielsweise der technische Vorteil erreicht, dass durch den Piezoschwinger eine hochfrequente Schwingfrequenz zur Herstellung des Flüssigkeitstropfennebels genutzt wird, bei der ein Anteil von Flüssigkeitstropfen der bevorzugten Größe besonders hoch ist.

In einer weiteren vorteilhaften Ausführungsform umfasst der Strömungserzeuger einen Ventilator oder eine Pumpe. Dadurch wird beispielsweise der technische Vorteil erreicht, dass der in dem Strömungsbehälter notwendige Luftstrom durch einfache bzw. kostengünstige Standardbauteile realisierbar ist.

In einer weiteren vorteilhaften Ausführungsform ist der Abstand zwischen dem Strömungsbehälterbereich und dem Flüssigkeitstropfenauslass proportional zu einem Verhältnis der ersten Zeitspanne der Beförderung der Flüssigkeitstropfen zu einer zweiten Zeitspanne des Absinkens eines Flüssigkeitstropfens bei einer vorbestimmten Tropfengröße entlang der vorbestimmten Absinkstrecke. Dadurch wird beispielsweise der technische Vorteil erreicht, dass der Strömungsbehälter in beliebiger Größe hergestellt werden kann. Wenn die angegebenen proportionalen Dimensionsverhältnisse eingehalten werden, ist somit jede gewünschte Größe eines Strömungsbehälters realisierbar.

In einer weiteren vorteilhaften Ausführungsform ist die vorbestimmte Größe des Flüssigkeitstropfens durch Veränderung einer Luftströmungsrate des Strömungserzeugers oder einer Frequenz des Tropfenerzeugers einstellbar. Dadurch wird beispielsweise der technische Vorteil erreicht, dass die Flüssigkeitstropfennebelerzeugung nicht auf eine bestimmte Flüssigkeitstropfengröße festgelegt ist, sondern mit den Parametern Luftströmungsrate des Strömungserzeugers und Schwingfrequenz des Tropfenerzeugers veränderbar ist.

In einer weiteren vorteilhaften Ausführungsform ist der Strömungsbehälter an der Oberseite über dem Strömungsbehälterbereich konkav ausgebildet, um an der Oberseite anhaftende Flüssigkeitstropfen in die Flüssigkeit des Strömungsbehälters zurückzuführen. Dadurch wird beispielsweise der technische Vorteil erreicht, dass diejenigen Flüssigkeitstropfen die durch den Tropfenerzeuger bis an die Oberseite des Strömungsbehälters geschleudert werden nicht dort verbleiben. In der Konsequenz wird dadurch der Flüssigkeitsverbrauch reduziert und regelmäßig notwendige Nachfüllintervalle von Flüssigkeit aufgrund des durch die Flüssigkeitstropfen verursachten Verbrauchs von Flüssigkeit werden verlängert.

In einer weiteren vorteilhaften Ausführungsform ist dem Flüssigkeitstropfenauslass ein mechanischer Tropfenfilter zur Selektion von Flüssigkeitstropfen vorbestimmter Größe nachgeschaltet. Dadurch wird beispielsweise der technische Vorteil erreicht, dass eine zusätzliche Selektion großer Flüssigkeitstropfen stattfindet, wenn die Flüssigkeitstropfen bereits den Strömungsbehälter verlassen haben.

In einer weiteren vorteilhaften Ausführungsform umfasst der mechanische Tropfenfilter eine Wandstruktur mit Luftströmungsumlenkungselementen wie Rippen oder Falten. Dadurch wird beispielsweise der technische Vorteil erreicht, dass mit einfachen und kostengünstigen Mitteln eine zusätzliche Selektion realisierbar ist, da Luftströmungsumlenkungselemente wie Rippen oder Falten einfach in eine Wandstruktur integrierbar sind. In der Konsequenz haften zu große Flüssigkeitstropfen, die mit dem Luftstrom in den Flüssigkeitstropfenauslass gelangt sind an den Luftströmungsumlenkungselementen des mechanischen Tropfenfilters an.

In einer weiteren vorteilhaften Ausführungsform weist der mechanische Tropfenfilter eine Struktur auf, die dazu eingerichtet ist, niedergeschlagene Flüssigkeitstropfen in die Flüssigkeit des Strömungsbehälters zurückzuführen. Dadurch wird beispielsweise der technische Vorteil erreicht, dass diejenigen Flüssigkeitstropfen die an der Wand des mechanischen Tropfenfilters anhaften nicht dort verbleiben. In der Konsequenz wird dadurch der Flüssigkeitsverbrauch reduziert und regelmäßig notwendige Nachfüllintervalle von Flüssigkeit aufgrund des durch die Flüssigkeitstropfennebelerzeugung verursachten Verbrauchs von Flüssigkeit werden verlängert.

In einer weiteren vorteilhaften Ausführungsform weist der Strömungsbehälter eine Hindernisvorrichtung auf, welche von der Luftströmung und den Flüssigkeitstropfen umströmt wird, wobei die Hindernisvorrichtung ausgebildet ist, an der Hindernisvorrichtung anhaftende Flüssigkeitstropfen in die Flüssigkeit des Strömungsbehälters zurückzuführen. Dadurch wird beispielsweise der technische Vorteil erreicht, dass eine weitere Selektion innerhalb des Strömungsbehälters stattfindet und den Flüssigkeitstropfennebel zusätzlich homogenisiert und somit einen zusätzlichen Betrag zu einer homogeneren Befeuchtung des Behandlungsgutes leistet. In der Konsequenz werden zu große Flüssigkeitstropfen unter Ausnutzung ihrer eigenen Trägheit aus dem Luftstrom dadurch entfernt, dass sie an der Hindernisvorrichtung anhaften und in die Flüssigkeit des Strömungsbehälters zurückfließen.

In einer weiteren vorteilhaften Ausführungsform umfasst die Hindernisvorrichtung einen Zylindervorhang oder eine Gitterstruktur. Dadurch wird beispielsweise der technische Vorteil erreicht, dass die Hindernisvorrichtung einfach zu realisieren ist und ggf. mehrere Stufen hintereinander umfasst.

In einer weiteren vorteilhaften Ausführungsform liegt die durch den Strömungserzeuger erzeugte Luftströmungsrate in einem Bereich zwischen 1 Liter und 30 Liter pro Minute und beträgt vorzugsweise 20 Liter pro Minute. Dadurch wird beispielsweise der technische Vorteil erreicht, dass einfache und kostengünstige Standardventilatoren oder Standardpumpen als Strömungserzeuger einsetzbar sind.

Weitere Ausführungsbeispiele der Erfindung sind in den Zeichnungen dargestellt und werden im Folgenden näher beschrieben.

Es zeigen:
- Fig. 1: eine schematische Querschnittsansicht eines Strömungsbehälters,
- Fig. 2: eine schematische Querschnittsansicht eines Strömungsbehälters mit vergrößertem horizontalem Abstand zwischen Strömungsbehälterbereich und Flüssigkeitstropfenauslass,
- Fig. 3a: eine schematische Ansicht eines Strömungsbehälters mit Hindernisvorrichtung in der Draufsicht, und
- Fig. 3b: eine schematische Querschnittsansicht eines Strömungsbehälters mit Hindernisvorrichtung.

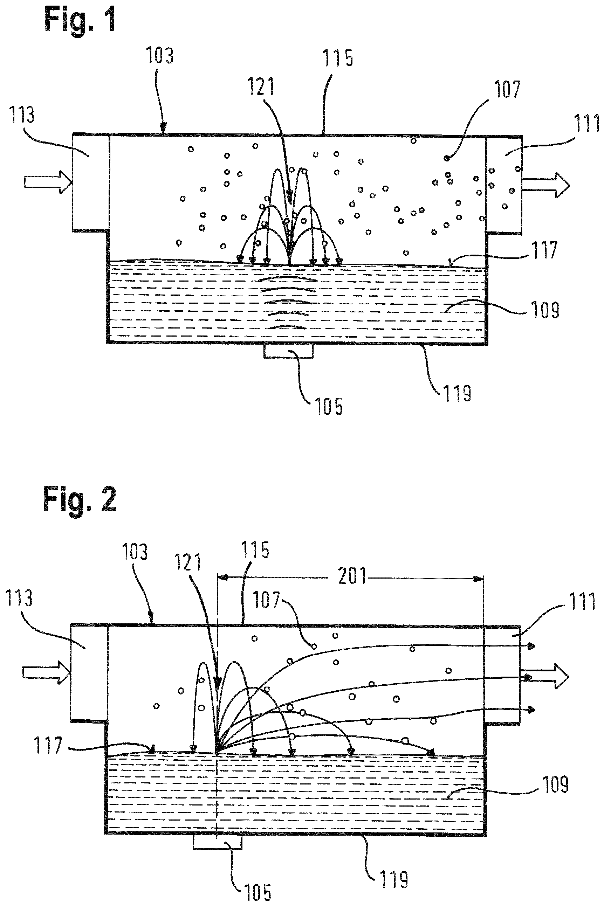

Fig. 1 zeigt eine schematische Querschnittsansicht eines Strömungsbehälters 103. In dem Strömungsbehälter 103 befindet sich eine Flüssigkeit 109 und an einem Boden 119 des Strömungsbehälters 103 ist ein Tropfenerzeuger 105, beispielsweise ein Piezoschwinger, angeordnet. Der Tropfenerzeuger 105 erzeugt eine hochfrequente mechanische Schwingung, die sich als longitudinale Welle durch die Flüssigkeit 109 bis zu einer Oberfläche 117 der Flüssigkeit 109 fortpflanzt und dort in einem Strömungsbehälterbereich 121 feine Flüssigkeitstropfen 107 aus der Oberfläche 117 herausschlägt.

Die Flüssigkeitstropfen 107 werden in einen Luftraum zwischen der Oberfläche 117 und einer Oberseite 115 des Strömungsbehälters 103 beispielsweise geschleudert und gelangen in den Strömungsbehälterbereich 121. Der Strömungsbehälter 103 umfasst einen Flüssigkeitstropfenauslass 111 und ein gegenüber dem Flüssigkeitstropfenauslass 111 angeordneten Strömungserzeuger 113. Der Strömungserzeuger 113 erzeugt einen Luftstrom, der im Wesentlichen parallel zur Oberfläche 117 der Flüssigkeit 109 in Richtung des Flüssigkeitstropfenauslasses 111 verläuft. Der erzeugte Luftstrom transportiert einen Teil der in dem Luftraum befindlichen Flüssigkeitstropfen 107 zu dem Flüssigkeitstropfenauslass 111, wo sie im Anschluss zu einem gewünschten Einsatzort in dem Haushaltsgerät 100 weitertransportiert werden. Der erzeugte Flüssigkeitstropfennebel umfasst gewöhnlich ein Spektrum von verschiedenen Flüssigkeitstropfengrößen. Große Flüssigkeitstropfen des Spektrums, die insbesondere eine Durchmessergröße von 10µm und mehr aufweisen, schlagen sich am Behandlungsgut und an Schaugläsern des Haushaltsgeräts 100 nieder.

Fig. 2 zeigt eine schematische Querschnittsansicht eines Strömungsbehälters 103 mit einem vergrößerten horizontalen Abstand 201 zwischen Strömungsbehälterbereich 121 und Flüssigkeitstropfenauslass 111. Auf eine wiederholte Beschreibung identischer Merkmale der vorausgehenden Figur wird verzichtet. Der Abstand 201 zwischen dem Strömungsbehälterbereich 121 und dem Flüssigkeitstropfenauslass 111 ist derart gewählt, dass eine durch den Luftstrom des Strömungserzeugers 113 verursachte Beförderungszeit der Flüssigkeitstropfen 107 zwischen dem Strömungsbehälterbereich 121 und dem Flüssigkeitstropfenauslass 111 gleich oder größer als eine Absinkzeit eines Flüssigkeitstropfens 107 einer bestimmten Tropfengröße von der Oberseite 115 des Strömungsbehälters 103 zurück in die Flüssigkeit 109 ist. Je größer der Durchmesser der Flüssigkeitstropfen 107 ist, desto kürzer ist, aufgrund ihres schnelleren Absinkens in die Flüssigkeit 109 des Strömungsbehälters 103, die Absinkzeit.

Daher erreichen nur Flüssigkeitstropfen 107 einer bestimmten Größe bzw. bis zu einem bestimmten maximalen Durchmesser den Flüssigkeitstropfenauslass 111 während die Flüssigkeitstropfen 107 ab dem bestimmten Durchmesser in die Flüssigkeit 109 absinken bevor sie den Flüssigkeitstropfenauslass 111 erreichen. Entscheidend wird dieser Effekt durch die Stokes'sche Reibung unterstützt, nach welcher sich kleinere Flüssigkeitstropfen 107 überproportional länger in der Luft halten können, als größere Flüssigkeitstropfen 107. Der Stokes'schen Reibung liegt die Annahme zugrunde, dass die Flüssigkeitstropfen 107 kugelförmig ausgebildet sind. So ergibt sich für die Flüssigkeitstropfen 107, dass deren Sinkgeschwindigkeit in Luft proportional zu dem Quadrat ihres Radius ist.

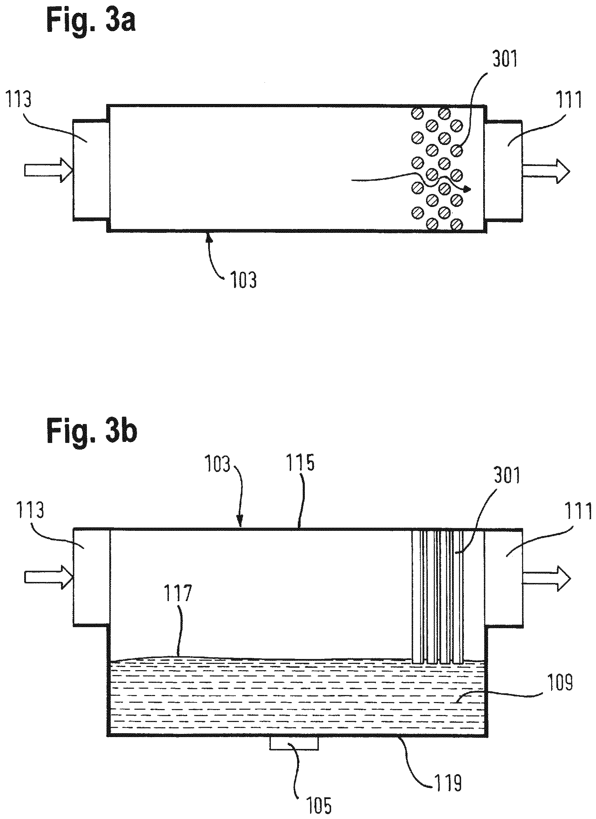

Fig. 3a zeigt eine schematische Ansicht eines Strömungsbehälters 103 mit einer Hindernisvorrichtung 301 in der Draufsicht. Auf eine wiederholte Beschreibung identischer Merkmale der vorausgehenden Figuren wird verzichtet. Der in dem Strömungsbehälter 103 angeordnete Strömungserzeuger 113 erzeugt einen Luftstrom, der im Wesentlichen parallel zur Oberfläche 117 der Flüssigkeit 109 in Richtung des Flüssigkeitstropfenauslasses 111 verläuft. Der erzeugte Luftstrom transportiert einen Teil der in dem Luftraum befindlichen Flüssigkeitstropfen 107 zu dem Flüssigkeitstropfenauslass 111, wo sie im Anschluss zu einem gewünschten Einsatzort in dem Haushaltsgerät 100 weitertransportiert werden. Zusätzlich umfasst der Strömungsbehälter 103 eine Hindernisvorrichtung 301, beispielsweise in Form eines einen Zylindervorhangs, welche von dem Luftstrom und den Flüssigkeitstropfen 107 umströmt wird. Dadurch, dass der Luftstrom einschließlich der Flüssigkeitstropfen 107 ggf. sehr enge Radien der Hindernisvorrichtung 301 passieren muss, erfahren Flüssigkeitstropfen 107 mit zunehmender Masse bzw. zunehmendem Durchmesser gegenüber Flüssigkeitstropfen 107 mit geringerer Masse eine stärkere Fliehkraft und werden deshalb beim Umströmen der Hindernisvorrichtung 301 weiter nach außen transportiert. Wenn sie dadurch auf die Hindernisvorrichtung 301 treffen, werden sie durch Adhäsion an dieser festgehalten, wodurch die Flüssigkeitstropfen 107 wieder zurück in die Flüssigkeit 109 des Strömungsbehälters 103 fließen. Alternativ kann die Hindernisvorrichtung 301 auch als Gitterstruktur oder durch angewinkelte Strömungsflächen ausgebildet sein.

Fig. 3b zeigt eine schematische Querschnittsansicht des Strömungsbehälters 103 mit Hindernisvorrichtung 301 aus Fig. 3a. Auf eine wiederholte Beschreibung identischer Merkmale der vorausgehenden Figuren wird verzichtet.

Sämtliche vorbenannte Ausführungsformen umfassen vorliegend einen Tropfenerzeuger 105 in Form eines Piezoschwingers. Die Schwingfrequenz des Piezoschwingers beträgt vorzugsweise 1,6 MHz, jedoch ist auch ein Piezoschwinger mit einer veränderlichen Schwingfrequenz denkbar. Dies hätte in Abstimmung mit einer veränderbaren Strömungsrate des Strömungserzeugers 113 den Vorteil, dass die erzeugte Flüssigkeitstropfengröße entsprechend verschiedener Anforderungen einstellbar ist. So würde beispielsweise eine Erhöhung der Schwingfrequenz des Piezoschwingers bei konstanter Strömungsrate des Strömungserzeugers 113 eine Reduzierung der Größe der Flüssigkeitstropfen in dem erzeugten Flüssigkeitstropfennebel zur Folge haben. Alternativ würde beispielsweise eine Erhöhung der Strömungsrate des Strömungserzeugers 113 bei konstanter Schwingfrequenz des Piezoschwingers zu einer Erhöhung der Größe der Flüssigkeitstropfen in dem erzeugten Flüssigkeitstropfennebel zur Folge haben.

Jeder der vorbenannten Ausführungsformen kann am Flüssigkeitstropfenauslass 111 ein mechanischer Tropfenfilter zur zusätzlichen Selektion von Flüssigkeitstropfen 107 nachgeschaltet sein. Diese mechanische Selektion von Flüssigkeitstropfen 107 kann durch eine Wandstruktur mit Luftströmungsumlenkungselementen wie Rippen oder Falten realisiert sein. Dies wäre insbesondere durch einen entsprechend geformten Schlauch oder ein entsprechend geformtes Rohr denkbar, wodurch der Flüssigkeitstropfennebel nachgeschaltet an einen gewünschten Einsatzort in dem Haushaltsgerät geleitet wird. Dies kann beispielsweise ein Arbeitsraum oder ein Laugenbehälter einer Wachmaschine sein. Zusätzlich oder alternativ umfasst der mechanische Tropfenfilter eine Bodenstruktur, die dazu eingerichtet ist die an der Wandstruktur niedergeschlagenen Flüssigkeitstropfen 107 in die Flüssigkeit 109 des Strömungsbehälters 103 zurückzuführen.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

### BEZUGSZEICHENLISTE

- 100: Haushaltsgerät
- 103: Strömungsbehälter
- 105: Tropfenerzeuger
- 107: Flüssigkeitstropfen
- 109: Flüssigkeit
- 111: Flüssigkeitstropfenauslass
- 113: Strömungserzeuger
- 115: Oberseite des Strömungsbehälters
- 117: Oberfläche der Flüssigkeit
- 119: Boden des Strömungsbehälters
- 121: Strömungsbehälterbereich
- 201: Abstand zwischen Strömungsbehälterbereich und Flüssigkeitstropfenauslass
- 301: Hindernisvorrichtung

## Patentansprüche

1. Haushaltsgerät (100) mit einem Strömungsbehälter (103), einem an dem Strömungsbehälter (103) angeordneten Tropfenerzeuger (105) zum Erzeugen von Flüssigkeitstropfen (107) aus einer Flüssigkeit (109) in einem Strömungsbehälterbereich (121) des Strömungsbehälters (103),- einem Flüssigkeitstropfenauslass (111) und einem gegenüber dem Flüssigkeitstropfenauslass (111) angeordneten Strömungserzeuger (113) zum Erzeugen einer Luftströmung zum Befördern der Flüssigkeitstropfen (107) zum Flüssigkeitstropfenauslass (111) **dadurch gekennzeichnet, dass** ein Abstand (201) zwischen dem Strömungsbehälterbereich (121) und dem Flüssigkeitstropfenauslass (111) derart gewählt ist, dass eine durch die Luftströmung des Strömungserzeugers (113) verursachte erste Zeitspanne der Beförderung von Flüssigkeitstropfen (107) zwischen dem Strömungsbehälterbereich (121) und dem Flüssigkeitstropfenauslass (111) gleich oder größer als eine zweite Zeitspanne des Absinkens eines Flüssigkeitstropfens (107) einer vorbestimmten Tropfengröße entlang einer vorbestimmten Absinkstrecke ist, so dass nur Flüssigkeitstropfen (107) einer bestimmten Größe bzw. bis zu einem bestimmten maximalen Durchmesser den Flüssigkeitstropfenauslass (111) erreichen.

2. Haushaltsgerät (100) nach Anspruch 1, **dadurch gekennzeichnet, dass** die vorbestimmte Absinkstrecke dem Abstand zwischen einer Oberseite des Strömungsbehälters (115) und einer Oberfläche (117) der Flüssigkeit (109) in dem Strömungsbehälter (103) entspricht.

3. Haushaltsgerät (100) nach einem der vorangehenden Ansprüche, **dadurch gekennzeichnet, dass** die vorbestimmte Größe der Flüssigkeitstropfen (107) in einem Bereich zwischen 1µm und 10µm liegt, vorzugsweise 5µm beträgt.

4. Haushaltsgerät (100) nach einem der vorangehenden Ansprüche, **dadurch gekennzeichnet, dass** der Tropfenerzeuger (105) in dem Strömungsbehälterbereich (121), insbesondere in oder am Boden (119) des Strömungsbehälters (103) angeordnet ist.

5. Haushaltsgerät (100) nach einem der vorangehenden Ansprüche, **dadurch gekennzeichnet, dass** der Tropfenerzeuger (105) ein Piezoschwinger mit einer Schwingfrequenz im Bereich zwischen 1MHz und 2 MHz ist, wobei die Schwingfrequenz vorzugsweise 1,6MHz beträgt.

6. Haushaltsgerät (100) nach einem der vorangehenden Ansprüche, **dadurch gekennzeichnet, dass** der Strömungserzeuger (113) einen Ventilator oder eine Pumpe umfasst.

7. Haushaltsgerät (100) nach einem der vorangehenden Ansprüche, **dadurch gekennzeichnet, dass** der Abstand (201) zwischen dem Strömungsbehälterbereich (121) und dem Flüssigkeitstropfenauslass (111) proportional zu einem Verhältnis der ersten Zeitspanne der Beförderung der Flüssigkeitstropfen (107) zu einer zweiten Zeitspanne des Absinkens eines Flüssigkeitstropfens (107) bei einer vorbestimmten Tropfengröße entlang der vorbestimmten Absinkstrecke ist.

8. Haushaltsgerät (100) nach einem der vorangehenden Ansprüche, **dadurch gekennzeichnet, dass** die vorbestimmte Größe des Flüssigkeitstropfens (107) durch Veränderung einer Luftströmungsrate des Strömungserzeugers (113) oder einer Frequenz des Tropfenerzeugers (105) einstellbar ist.

9. Haushaltsgerät (100) nach einem der vorangehenden Ansprüche, **dadurch gekennzeichnet, dass** der Strömungsbehälter (103) an der Oberseite (115) über dem Tropfenerzeuger (105) konkav ausgebildet ist, um an der Oberseite (115) anhaftende Flüssigkeitstropfen (107) in die Flüssigkeit (109) des Strömungsbehälters (103) zurückzuführen.

10. Haushaltsgerät (100) nach einem der vorangehenden Ansprüche, **dadurch gekennzeichnet, dass** dem Flüssigkeitstropfenauslass (111) ein mechanischer Tropfenfilter zur Selektion von Flüssigkeitstropfen (107) vorbestimmter Größe nachgeschaltet ist.

11. Haushaltsgerät (100) nach Anspruch 10, **dadurch gekennzeichnet, dass** der mechanische Tropfenfilter eine Wandstruktur mit Luftströmungsumlenkungselementen wie Rippen oder Falten umfasst.

12. Haushaltsgerät (100) nach Anspruch 10 oder 11, **dadurch gekennzeichnet, dass** der mechanische Tropfenfilter eine Struktur aufweist, die dazu eingerichtet ist niedergeschlagenen Flüssigkeitstropfen (107) in die Flüssigkeit (109) des Strömungsbehälters (103) zurückzuführen.

13. Haushaltsgerät (100) nach einem der vorangehenden Ansprüche, **dadurch gekennzeichnet, dass** der Strömungsbehälter (103) eine Hindernisvorrichtung (301) aufweist, welche von der Luftströmung und den Flüssigkeitstropfen (107) umströmt wird, wobei die Hindernisvorrichtung (301) ausgebildet ist, an der Hindernisvorrichtung (301) anhaftende Flüssigkeitstropfen (107) in die Flüssigkeit (109) des Strömungsbehälters (103) zurückzuführen.

14. Haushaltsgerät (100) nach einem der vorangehenden Ansprüche, **dadurch gekennzeichnet, dass** die Hindernisvorrichtung (301) einen Zylindervorhang oder eine Gitterstruktur umfasst.

15. Haushaltsgerät (100) nach einem der vorangehenden Ansprüche, **dadurch gekennzeichnet, dass** die durch den Strömungserzeuger (113) erzeugte Luftströmungsrate in einem Bereich zwischen 1 Liter und 30 Liter pro Minute liegt, vorzugsweise 20 Liter pro Minute beträgt.

## Claims

1. Domestic appliance (100) comprising a flow container (103), a drop generator (105) arranged on the flow container (103) for generating liquid drops (107) from a liquid (109) in a flow container region (121) of the flow container (103), a liquid drop outlet (111) and a flow generator (113) arranged opposite the liquid drop outlet (111) for generating an air flow for conveying the liquid drops (107) to the liquid drop outlet (111), **characterised in that** a distance (201) between the flow container region (121) and the liquid drop outlet (111) is selected in such a way that a first time span - caused by the air flow of the flow generator (113) - of the conveyance of liquid drops (107) between the flow container region (121) and the liquid drop outlet (111) is equal to or greater than a second time span of the falling of a liquid drop (107) of a predetermined drop size along a predetermined falling route, such that only liquid drops (107) of a particular size or up to a particular maximum diameter reach the liquid drop outlet (111).

2. Domestic appliance (100) according to claim 1, **characterised in that** the predetermined falling route corresponds to the distance between an upper side of the flow container (115) and an upper surface (117) of the liquid (109) in the flow container (103).

3. Domestic appliance (100) according to one of the preceding claims, **characterised in that** the predetermined size of the liquid drops (107) lies in a range between 1 µm and 10 µm and is preferably 5 µm.

4. Domestic appliance (100) according to one of the preceding claims, **characterised in that** the drop generator (105) is arranged in the flow container region (121), in particular in or on the base (119) of the flow container (103).

5. Domestic appliance (100) according to one of the preceding claims, **characterised in that** the drop generator (105) is a piezoelectric oscillator having an oscillation frequency in the range between 1 MHz and 2 MHz, wherein the oscillation frequency is preferably 1.6 MHz.

6. Domestic appliance (100) according to one of the preceding claims, **characterised in that** the flow generator (113) comprises a fan or a pump.

7. Domestic appliance (100) according to one of the preceding claims, **characterised in that** the distance (201) between the flow container region (121) and the liquid drop outlet (111) is proportional to a ratio of the first time span of the conveyance of the liquid drops (107) to a second time span of the falling of a liquid drop (107) at a predetermined drop size along the predetermined falling route.

8. Domestic appliance (100) according to one of the preceding claims, **characterised in that** the predetermined size of the liquid drop (107) can be set by changing an air flow rate of the flow generator (113) or a frequency of the drop generator (105).

9. Domestic appliance (100) according to one of the preceding claims, **characterised in that** the flow container (103) is embodied in concave fashion on the upper side (115) above the drop generator (105) in order to return liquid drops (107) adhering to the upper side (115) into the liquid (109) of the flow container (103).

10. Domestic appliance (100) according to one of the preceding claims, **characterised in that** a mechanical drop filter is connected downstream of the liquid drop outlet (111) in order to select liquid drops (107) of a predetermined size.

11. Domestic appliance (100) according to claim 10, **characterised in that** the mechanical drop filter comprises a wall structure having air flow deflection elements such as ribs or folds.

12. Domestic appliance (100) according to claim 10 or 11, **characterised in that** the mechanical drop filter exhibits a structure which is designed in order to return deposited liquid drops (107) into the liquid (109) of the flow container (103).

13. Domestic appliance (100) according to one of the preceding claims, **characterised in that** the flow container (103) has a barrier device (301) around which the air flow and the liquid drops (107) flow, wherein the barrier device (301) is designed in order to return liquid drops (107) adhering to the barrier device (301) into the liquid (109) of the flow container (103).

14. Domestic appliance (100) according to one of the preceding claims, **characterised in that** the barrier device (301) comprises a cylinder curtain or a grid structure.

15. Domestic appliance (100) according to one of the preceding claims, **characterised in that** the air flow rate generated by the flow generator (113) lies in a range between 1 litre and 30 litres per minute and is preferably 20 litres per minute.

## Revendications

1. Appareil ménager (100) comprenant un réservoir d'écoulement (103), un générateur de gouttes (105) disposé sur le réservoir d'écoulement (103), destiné à générer des gouttes de liquide (107) à partir d'un liquide (109) dans une zone de réservoir d'écoulement (121) du réservoir d'écoulement (103), comprenant une sortie de gouttes de liquide (111) et un générateur de flux (113) disposé en vis-à-vis de la sortie de gouttes de liquide (111), destiné à générer un flux d'air pour transporter les gouttes de liquide (107) vers la sortie de gouttes de liquide (111), **caractérisé en ce qu'**un écart (201) entre la zone de réservoir d'écoulement (121) et la sortie de gouttes de liquide (111) est sélectionné de manière à ce qu'une première période de temps, causée par le flux d'air du générateur de flux (113), du transport de gouttes de liquide (107) entre la zone de réservoir d'écoulement (121) et la sortie de gouttes de liquide (111) soit égale ou supérieure à une deuxième période de temps de la chute d'une goutte de liquide (107) d'une taille de goutte prédéterminée le long d'une trajectoire de chute prédéterminée, de sorte que seulement des gouttes de liquide (107) d'une taille déterminée, resp. jusqu'à un diamètre maximal déterminé, atteignent la sortie de gouttes de liquide (111).

2. Appareil ménager (100) selon la revendication 1, **caractérisé en ce que** la trajectoire de chute prédéterminée correspond à l'écart entre un côté supérieur du réservoir d'écoulement (115) et une surface (117) du liquide (109) dans le réservoir d'écoulement (103).

3. Appareil ménager (100) selon l'une quelconque des revendications précédentes, **caractérisé en ce que** la taille prédéterminée des gouttes de liquide (107) est située dans une plage comprise entre 1 µm et 10 µm, de préférence **en ce qu'**elle est de 5 µm.

4. Appareil ménager (100) selon l'une quelconque des revendications précédentes, **caractérisé en ce que** le générateur de gouttes (105) est disposé dans la zone de réservoir d'écoulement (121), notamment dans ou sur le fond (119) du réservoir d'écoulement (103).

5. Appareil ménager (100) selon l'une quelconque des revendications précédentes, **caractérisé en ce que** le générateur de gouttes (105) est un oscillateur piézoélectrique ayant une fréquence d'oscillation située dans la plage comprise entre 1 MHz et 2 MHz, la fréquence d'oscillation étant de préférence de 1,6 MHz.

6. Appareil ménager (100) selon l'une quelconque des revendications précédentes, **caractérisé en ce que** le générateur de flux (113) comprend un ventilateur ou une pompe.

7. Appareil ménager (100) selon l'une quelconque des revendications précédentes, **caractérisé en ce que** l'écart (201) entre la zone de réservoir d'écoulement (121) et la sortie de gouttes de liquide (111) est proportionnel à un rapport entre la première période de temps du transport des gouttes de liquide (107) et une deuxième période de temps de la chute d'une goutte de liquide (107), dans le cas d'une taille de gouttes prédéterminée, le long de la trajectoire de chute prédéterminée.

8. Appareil ménager (100) selon l'une quelconque des revendications précédentes, **caractérisé en ce que** la taille prédéterminée de la goutte de liquide (107) est réglable par modification d'un taux de flux d'air du générateur de flux (113) ou d'une fréquence du générateur de gouttes (105).

9. Appareil ménager (100) selon l'une quelconque des revendications précédentes, **caractérisé en ce que** le réservoir d'écoulement (103) est réalisé de manière concave sur le côté supérieur (115) au-dessus du générateur de gouttes (105) afin de ramener dans le liquide (109) du réservoir d'écoulement (103) des gouttes de liquide (107) adhérant sur le côté supérieur (115).

10. Appareil ménager (100) selon l'une quelconque des revendications précédentes, **caractérisé en ce qu'**un filtre mécanique de gouttes est installé en aval de la sortie de gouttes de liquide (111) pour la sélection de gouttes de liquide (107) de taille prédéterminée.

11. Appareil ménager (100) selon la revendication 10, **caractérisé en ce que** le filtre mécanique de gouttes comprend une structure murale dotée d'éléments de déviation de flux d'air, telle que des nervures ou des plis.

12. Appareil ménager (100) selon la revendication 10 ou 11, **caractérisé en ce que** le filtre mécanique de gouttes présente une structure qui est configurée pour ramener dans le liquide (109) du réservoir d'écoulement (103) les gouttes de liquide (107) précipitées.

13. Appareil ménager (100) selon l'une quelconque des revendications précédentes, **caractérisé en ce que** le réservoir d'écoulement (103) présente un dispositif barrière (301) qui est contourné par le flux d'air et les gouttes de liquide (107), le dispositif barrière (301) étant réalisé pour ramener dans le liquide (109) du réservoir d'écoulement (103) les gouttes de liquide (107) adhérant au dispositif barrière (301).

14. Appareil ménager (100) selon l'une quelconque des revendications précédentes, **caractérisé en ce que** le dispositif barrière (301) comprend un rideau cylindrique ou une structure en grille.

15. Appareil ménager (100) selon l'une quelconque des revendications précédentes, **caractérisé en ce que** le taux de flux d'air généré par le générateur de flux (113) est situé dans une plage comprise entre 1 litre et 30 litres par minute, de préférence **en ce qu'**il est de 20 litres par minute.
